# EUROPÄISCHE PATENTSCHRIFT

(11) **EP 0 910 318 B1**
(45) Veröffentlichungstag und Bekanntmachung des Hinweises auf die Patenterteilung: **31.05.2000**
(21) Anmeldenummer: 98914864.8
(22) Anmeldetag: 04.03.1998
(51) Int. Cl.: A61F 2/60

(54) **FEDEREINRICHTUNG FÜR EINE BEINPROTHESE**
LEG PROSTHESIS DAMPING DEVICE
DISPOSITIF D'AMORTISSEMENT POUR UNE PROTHESE DE JAMBE

(30) Priorität: 05.03.1997 DE 19709006
(43) Veröffentlichungstag der Anmeldung: 28.04.1999
(73) Patentinhaber: BIEDERMANN MOTECH GmbH, 78054 Villingen-Schwenningen (DE)
(72) Erfinder: BIEDERMANN, Lutz, 78048 VS-Villingen (DE)
(74) Vertreter: Prüfer, Lutz H., Dipl.-Phys.
(86) Internationale Anmeldenummer: EP9801210
(87) Internationale Veröffentlichungsnummer: WO9838951

(56) Entgegenhaltungen:
- FR-A- 371 291

## Beschreibung

Die Erfindung betrifft eine Federeinrichtung für eine Beinprothese.

Beim Laufen mit einer Beinprothese wird bei jedem Schritt ein erheblicher Stoß auf die Hüfte und das Kreuz geleitet. Als Dämpfung dieses Stoßes ist es bekannt, zwischen den beiden mit dem Stumpf bzw. dem Fuß zu verbindenden Abschnitten eine Dämpfung vorzusehen. Eine solche Dämpfung umfaßt zwei ineinander passende Hülsen, die relativ zueinander in axialer Richtung bewegbar sind, und einen im Inneren der beiden Hülsen vorgesehenen Kompressionsring aus einem Kunststoff, der in Normalstellung entspannt ist und sich bei dem Auftreten zusammendrückt und den Stoß aufnimmt. Bei Entlastung richtet er sich wieder auf.

Eine Feder für eine Prothese ist aus der DE 295 16 455 U1 und aus der DE 91 12 005 U1 bekannt.

Die Merkmale des Oberbegriffs des Anspruchs 1 sind aus der FR-A-371 291 bekannt.

Aufgabe der Erfindung ist es, eine Federeinrichtung zu schaffen, mit der die Ablaufbewegung des Patienten besser unterstützt werden soll und auch eine individuelle Einstellung bzw. Anpassung für den Patienten möglich ist.

Diese Aufgabe wird durch die in dem Patentanspruch 1 beschriebene Federeinrichtung gelöst. Diese Federeinrichtung erreicht, daß nicht nur eine Dämpfung beim Fersenauftritt auftritt, sondern auch eine Kraft für die Rückführung beim Zehenabstoß auftritt. Ferner ist die Einrichtung kostengünstig herstellbar und damit für einen breiten Einsatz geeignet.

Weiterbildungen der Erfindung sind in den Unteransprüchen gekennzeichnet. Weitere Merkmale und Zweckmäßigkeiten der Erfindung ergeben sich aus der Beschreibung von Ausführungsbeispielen anhand der Figuren. Von den Figuren zeigen:
- Fig. 1: eine Seitenansicht einer Prothese mit der Federeinrichtung;
- Fig. 2: eine Schnittdarstellung durch eine erste Ausführungsform der Federeinrichtung;
- Fig. 3: eine Schnittdarstellung;
- Fig. 4: eine Seitenansicht der beiden Ausführungsformen;
- Fig. 5: eine der Darstellung in Fig. 2 entsprechende Darstellung; und
- Fig. 6: eine der Darstellung in Fig. 3 entsprechende Darstellung.

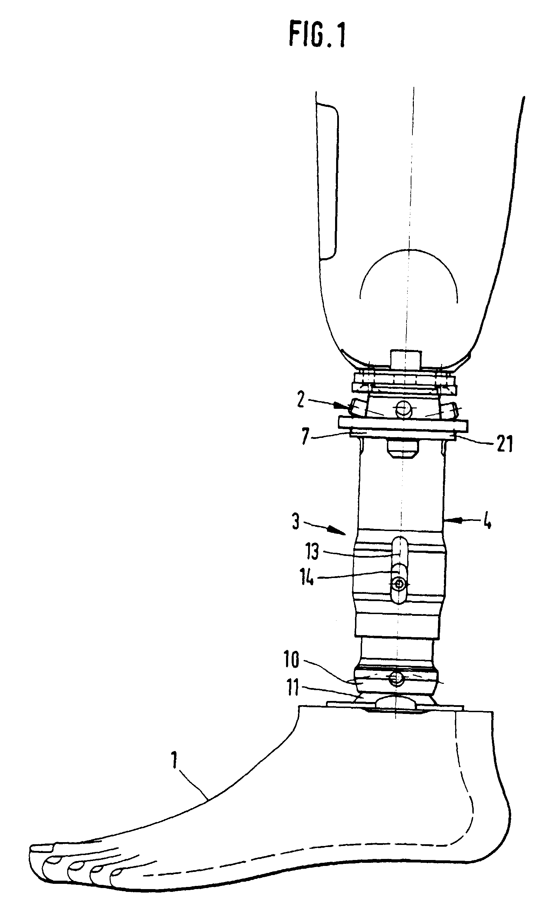

Wie in Fig. 1 gezeigt ist, umfaßt eine Beinprothese einen einen Fußteil 1 und einen schematisch angedeuteten Unterschenkel oder Oberschenkel aufnehmenden Anschlußteil 2 sowie einen diese beiden Teile miteinander verbindenden rohrförmigen Abschnitt 3.

Bei der erfindungsgemäßen Ausführungsform ist der rohrförmige Abschnitt als eine Federeinrichtung ausgebildet. Diese weist ein rohrförmiges Gehäuse 4 auf. Das Gehäuse wird gebildet aus einer äußeren Hülse 5 und einer mit einem Abschnitt in diese hineinragende inneren Hülse 6. Die äußere Hülse 5 ist in ihrem Inneren zylindrisch ausgebildet. An ihrem einen Ende weist sie einen Boden 7 auf, während sie an ihrem dem Boden gegenüberliegenden Ende offen ausgebildet ist. Die innere Hülse 6 ist an ihrem dem Boden 7 der äußeren Hülse zugewandten freien Ende 8 offen und besitzt in einem vorbestimmten Abstand von dem freien Ende einen Zwischenboden 9. Das dem freien Ende 8 abgewandte zweite Ende 10 der inneren Hülse 6 ist so ausgebildet, daß es mit einem Koppelelement 11 des Fußteiles 1 in üblicher Weise verbindbar ist. Zwischen dem Boden 7 und dem Zwischenboden 9 ist eine Druckfeder 12 angeordnet. Die innere Hülse 6 ist auf ihrer Außenfläche relativ zur inneren Form der äußeren Hülse 5 ebenfalls zylindrisch ausgebildet, so daß die innere Hülse mit einem vorbestimmten Abschnitt in das Innere der äußeren Hülse hineinführbar und in der äußeren Hülse in axialer Richtung hin und her bewegbar ist.

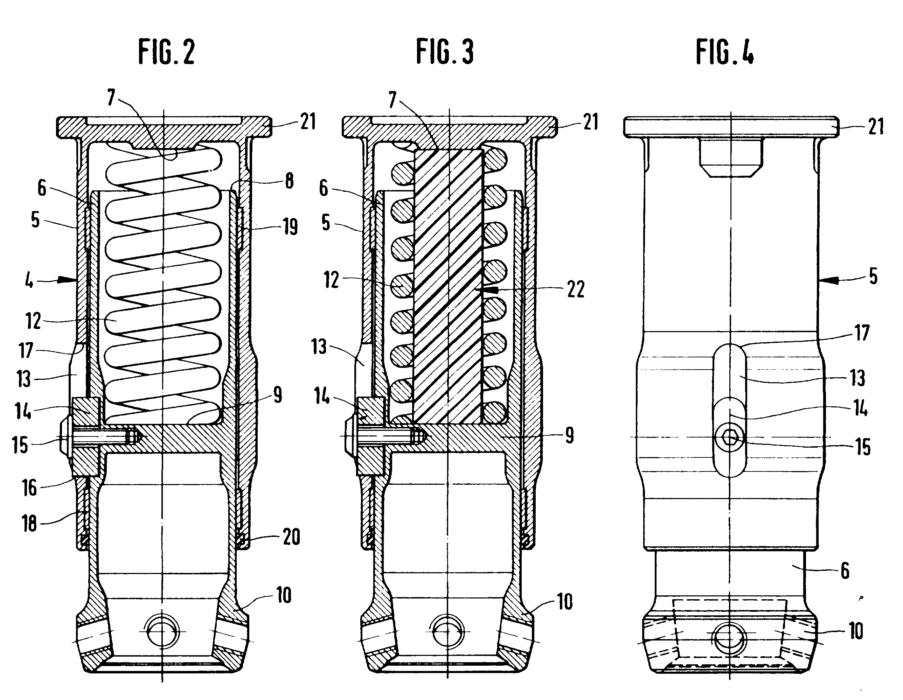

Wie am besten aus den Figuren 2 und 4 ersichtlich ist, weist die äußere Hülse 5 einen sich in axialer Richtung erstreckenden Schlitz 13 vorbestimmter Länge auf. Die innere Hülse 5 weist eine als Nutstein ausgebildete Paßfeder 14 auf, die mittels einer Schraube 15, welche im Bereich des Zwischenbodens in radialer Richtung in die innere Hülse eingeschraubt ist, befestigt ist. Die Länge des Schlitzes 13 und die in axiale Richtung sich erstreckende Länge des Nutsteines 14 sind so gewählt, daß die beiden Hülsen zwischen der in den Figuren 2 und 4 gezeigten expandierten Stellung, bei der der Nutstein am unteren Rand 16 des Schlitzes anschlägt, und in einer zusammengepreßten Stellung, in der der Nutstein am oberen Rand 17 des Schlitzes anschlägt, hin und her bewegbar sind. Der Abstand des Zwischenbodens 9 von dem freien Ende 8 ist so gewählt, daß die zwischen dem Boden 7 und dem Zwischenboden 9 angeordnete Druckfeder 12 in der in Fig. 2 gezeigten expandierten Stellung eine vorbestimmte Vorspannung aufweist.

Wie aus Fig. 2 ersichtlich ist, weist die äußere Hülse nahe bei ihrem dem Boden 7 gegenüberliegenden freien Ende eine ringbandartige Gleitführung 18 auf. Ferner weist die äußere Hülse 5 in einer Position, die in der expandierten Stellung nahe am freien Ende 8 der inneren Hülse 6 und dieser benachbart ist, eine entsprechende zweite ringbandförmig ausgebildete Gleitführung 19 auf. Die Gleitführungen 18, 19 sind jeweils aus Teflon oder aus einem Material mit vergleichbaren Eigenschaften ausgebildet. Am freien Ende der äußeren Hülse 5 ist ferner eine O-Ring-Dichtung 20 angeordnet, die dazu dient, das Eintreten von Staub oder ähnlichem in die Federeinrichtung zu verhindern.

Durch das Vorsehen der beiden Gleitführungen 18, 19 wird das bei bekannten Dämpfungselementen beobachtete Stick-Shift-Phänomen beim Ein- und Ausfedern vermieden.

Die in den Figuren 2 und 4 gezeigte Federvorrichtung ist mit ihrem zweiten Ende 10 mit dem Fußteil 1 und über ihren diesem Ende gegenüberliegenden Flansch 21 mit dem Stumpf-Anschlußteil 2 verbunden. Im Betrieb erfolgt beim Auftreten eine Dämpfung über die Feder 12. Durch die Federvorspannung wird auch erreicht, daß nicht nur die Dämpfung erfolgt, sondern daß die Federkraft eine Rückführung des Federelementes in die expandierte Stellung bewirkt. Die Führung der Paßfeder 14 in dem Schlitz 13 bewirkt, daß jede Drehbewegung zwischen Fuß und Stumpf vermieden wird. Das Ineinandergleiten der ersten und zweiten Hülse wirkt einer Kippbewegung entgegen. Dadurch wird mit einer konstruktiv einfachen Federvorrichtung die Benutzung einer Beinprothese erheblich komfortabler.

Die in Fig. 3 gezeigte abgewandelte Ausführungsform der Federvorrichtung unterscheidet sich von der zuerst beschriebenen Ausführungsform lediglich dadurch, daß zusätzlich zu der Druckfeder 12 noch ein Dämpfungselement 22 vorgesehen ist. Alle übrigen Merkmale der Federvorrichtung stimmen mit der zuerst beschriebenen Ausführungsform überein.

Das Dämpfungselement 22 ist als ein Zylinder ausgebildet, dessen Länge gleich dem Abstand der dem Zwischenboden 9 zugewandten Fläche des Bodens 7 und der den Boden 7 zugewandten Fläche des Zwischenbodens 9 in dem in Fig. 3 gezeigten expandierten Zustand ist. Der Durchmesser des Zylinders ist so ausgebildet, daß er im wesentlichen gleich dem inneren Durchmesser der Druckfeder 12 ist und somit im Inneren von der Druckfeder geführt ist. Das Dämpfungselement 22 ist vorzugsweise aus Polyurethan oder einem Material mit vergleichbaren Dämpfungseigenschaften gebildet.

Im Betrieb trägt das Dämpfungselement 22 zur Dämpfung des Schrittes bei, während die Feder 12 in der bereits beschriebenen Weise sowohl zur Dämpfung als auch zur Rückführung des Federelementes in die gezeigte expandierte Stellung beiträgt. Die Kombination von Dämpfungs- und Federelement erlaubt außerdem die Wahl der Federkennlinie angepaßt an die Laufbedürfnisse des Prothesenträgers.

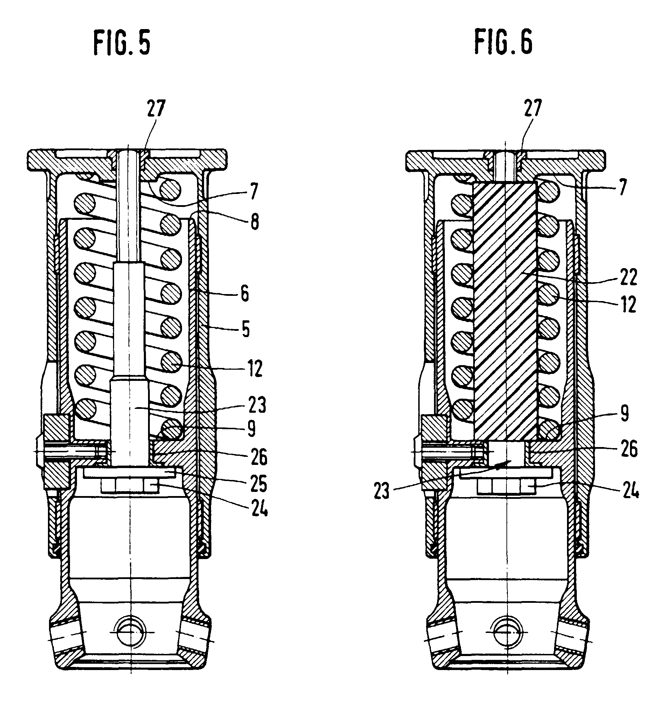

Die in Fig. 5 gezeigte Ausführungsform stimmt zunächst in allen Merkmalen mit der in Fig. 2 gezeigten Ausführungsform überein. Zum Erreichen einer Vorspannung der Feder 12 ist zusätzlich eine Einstellschraube 23 vorgesehen. Diese ist koaxial zu den beiden Hülsen 5, 6 angeordnet. Mit ihrem Kopf 24 und einer Unterlegscheibe 25 ruht sie auf der äußeren Seite des Zwischenbodens 9 und ist durch eine mit einer Dichtung 26 abgedichtete Bohrung geführt. Ihr freies Ende reicht durch eine ebenfalls koaxiale Bohrung in dem Boden 7 und ist mit einer auf der Außenseite des Bodens 7 ruhenden Mutter 27 in Eingriff.

Die in Fig. 6 gezeigte Ausführungsform entspricht zunächst in allen Merkmalen der in Fig. 3 gezeigten Ausführungsform. Zusätzlich ist eine Einstellschraube 23 vorgesehen, die in allen Einzelheiten der in Fig. 5 gezeigten Ausführungsform mit Kopf und Mutter entspricht und die durch eine konzentrische Bohrung des Dämpfungselementes 22 hindurchgeführt ist.

Im Betrieb wird die Vorspannung der Feder 12 bzw. 12 und 22 mittels der Einstellschraube 23 in Abhängigkeit vom Gewicht und dem Aktivitätsgrad des Patienten eingestellt.

## Patentansprüche

1. Federeinrichtung für eine Beinprothese mit einem rohrförmigen Gehäuse aus einer äußeren Hülse (5), die an ihrem einen Ende einen Boden (7) aufweist und an ihrem anderen Ende offen ist,
und einer inneren Hülse (6), deren Außenabmessungen wenigstens in einem Abschnitt so bemessen sind, daß die innere Hülse (6) und die äußerer Hülse (5) in axialer Richtung relativ zueinander verschiebbar sind, und die mit wenigstens einem Abschnitt im Inneren der äußeren Hülse (5) angeordnet ist, und
die an ihrem dem Boden (7) zugewandten freien Ende (8) offen ist und in einem Abstand von dem freien Ende (8) ein Widerlager (9) für eine Druckfeder aufweist,
und mit einer zwischen dem Boden (7) und dem Widerlager (9) in vorgespannter Position angeordneten Druckfeder (12), dadurch gekennzeichnet, daß
die äußere Hülse (5) am bodenseitigen Ende (7) und die innere Hülse (5) an ihrem dem freien Ende (8) abgewandten Ende jeweils Elemente (10, 21) zum Verbinden mit einem Stumpfverbindungsteil (2) bzw. mit dem Fußteil (11, 1) aufweisen, wobei die beiden Hülsen (5, 6) im Bereich ihrer jeweiligen freien Enden Gleitelemente (18, 19) aufweisen.

2. Federvorrichtung nach Anspruch 1, dadurch gekennzeichnet, daß die beiden Hülsen (5, 6) so ineinander geführt sind, daß eine Bewegung in Drehrichtung verhindert ist.

3. Federvorrichtung nach Anspruch 2, dadurch gekennzeichnet, daß eine der Hülsen (5) einen sich in axialer Richtung erstreckenden Schlitz (13) und die andere Hülse (6) ein in den Schlitz (13) eingreifendes Führungselement (14) aufweisen.

4. Federvorrichtung nach einem der Ansprüche 1 bis 3, dadurch gekennzeichnet, daß wenigstens eines der Gleitelemente (18, 19) als ringförmiges Führungsband ausgebildet ist.

5. Federvorrichtung nach einem der Ansprüche 1 bis 4, dadurch gekennzeichnet, daß ein Anschlag (16, 17) zur Begrenzung der Relativbewegung der beiden Hülsen (5, 6) zwischen einer ersten und einer zweiten Stellung vorgesehen ist.

6. Federvorrichtung nach einem der Ansprüche 3 bis 5, dadurch gekennzeichnet, daß der Schlitz (13) in seiner Länge und das Führungselement (14) so dimensioniert sind, daß der jeweilige Anschlag (16, 17) der ersten bzw. zweiten Stellung entspricht.

7. Federvorrichtung nach einem der Ansprüche 1 bis 6, dadurch gekennzeichnet, daß zum Bewirken und Einstellen der Vorspannung ein Einstellelement (23) vorgesehen ist.

8. Federvorrichtung nach Anspruch 7, dadurch gekennzeichnet, daß das Einstellelement als eine Einstellschraube (23) ausgebildet ist.

## Claims

1. Damping device for a leg prosthesis with tubular casing consisting of an outer sleeve (5) which has a base (7) at one end and is open at the other end,
and an inner sleeve (6), of which the external dimensions at least in one portion are such that the inner sleeve (6) and the outer sleeve (5) can be displaced in the axial direction relative to one another, and which is disposed with at least one portion in the interior of the outer sleeve (5), and
which is open at its free end (8) facing the base (7) and has an abutment (9) for a pressure spring at a distance from the free end (8),
and with a pressure spring (12) disposed between the base (7) and the abutment (9) in the initially tensioned position,
characterised in that
the outer sleeve (5) at the base end (7) and the inner sleeve (5) at its end remote from the free end (8) each have elements (10, 21) for connection to a stump connecting part (2) or to the foot part (11, 1) respectively, the two sleeves (5, 6) having slide elements (18, 19) in the region of their respective free ends.

2. Damping device according to Claim 1, characterised in that the two sleeves (5, 6) are guided in one another in such a way that a movement in the rotary direction is prevented.

3. Damping device according to Claim 2, characterised in that one of the sleeves (5) has a slot (13) extending in the axial direction and the other sleeve (6) has a guide element (14) engaging in the slot (13).

4. Damping device according to one of Claims 1 to 3, characterised in that at least one of the slide elements (18, 19) is constructed as an annular guide band.

5. Damping device according to one of Claims 1 to 4, characterised in that a stop (16, 17) is provided in order to limit the relative movement of the two sleeves (5, 6) between a first and a second position.

6. Damping device according to one of Claims 3 to 5, characterised in that the length of the slot (13) and the guide element (14) are so dimensioned that the respective stop (16, 17) corresponds to the first or second position respectively.

7. Damping device according to one of Claims 1 to 6, characterised in that an adjusting element (23) is provided in order to effect and to adjust the initial tension.

8. Damping device according to Claim 7, characterised in that the adjusting element is constructed as an adjusting screw (23).

## Revendications

1. Dispositif amortisseur pour une prothèse de jambe à un carter tubulaire formé par un tube extérieur (5), qui comprend un fond (7) de sa première extrémité et qui est ouvert à son autre extrémité,
et formé par un tube intérieur (6) dont les dimensions extérieures sont si dimensionnées, au moins à un tronçon, que ledit tube intérieur (6) et ledit tube extérieur (5) soient déplaçables en sens axial l'un relativement à l'autre, et qui est disposé par au moins un tronçon à l'intérieur dudit tube extérieur (5), et
qui est ouvert à son extrémité libre (8) en regard dudit fond (7) et qui comprend une butée (9) pour un ressort à pression à une distance de ladite extrémité libre (8),
et à un ressort à pression (12) disposé en une position en précontrainte entre ledit fond (7) et ladite butée (9),
caractérisé en ce que ledit tube extérieur (5), à son extrémité du côté du fond (7), et ledit tube intérieur (5), à son extrémité opposée de son extrémité libre (8), comprennent chacun des éléments respectifs (10, 21) à leur raccord à un élément (2) de raccord au moignon ou respectivement à l'élément de pied (11, 1), lesdits deux tubes (5, 6) comprenant des éléments de glissement (18, 19) dans la zone de leurs extrémités libres respectives.

2. Dispositif amortisseur selon la revendication 1, caractérisé en ce que lesdits deux tubes (5, 6) sont guidés l'un dans l'autre de façon à empêcher un mouvement en sens de rotation.

3. Dispositif amortisseur selon la revendication 2, caractérisé en ce qu'un (5) desdits tubes présente une fente (13), qui s'étend en sens axial, pendant que l'autre tube (6) présente un élément de guidage (14) engagé dans ladite fente (13).

4. Dispositif amortisseur selon une quelconque des revendications 1 à 3, caractérisé en ce qu'au moins un desdits éléments de glissement (18, 19) est configuré sous forme d'un feuillard de guidage annulaire.

5. Dispositif amortisseur selon une quelconque des revendications 1 à 4, caractérisé en ce qu'une butée (16, 17) est disposée à limiter le mouvement relatif desdits deux tubes (5, 6) entre une première position et une deuxième position.

6. Dispositif amortisseur selon une quelconque des revendications 3 à 5, caractérisé en ce que la longueur de ladite fente (13) et ledit élément de guidage (14) sont si dimensionnés que la butée respective (16, 17) corresponde à la première ou respectivement à la deuxième position.

7. Dispositif amortisseur selon une quelconque des revendications 1 à 6, caractérisé en ce qu'un élément de réglage (23) est disposé à créer et régler la précontrainte.

8. Dispositif amortisseur selon la revendication 7, caractérisé en ce que ledit élément de réglage est configuré sous forme d'une vis d'ajustage.
